# EUROPÄISCHE PATENTANMELDUNG

(11) **EP 1 149 584 A1**
(43) Veröffentlichungstag der Anmeldung: **31.10.2001**
(21) Anmeldenummer: 01108769.9
(22) Anmeldetag: 06.04.2001
(51) Int. Cl.: A61K 38/42, A61P 35/00

(54) **Verwendung von Photofrin als Radiosensitizer**

(30) Priorität: 28.04.2000 DE 10020935
(71) Anmelder: Hofstetter, Alfons, Prof.Dr.med., 82008 Unterhaching (DE); Dühmke, Eckhart, Prof., 82327 Tutzing (DE); Schaffer, Moshe, 81377 Munich (DE); Jori, Giulio, 35100 Padova (IT); Corti, Luigi, Prof., 35152 Padua (IT)
(72) Erfinder: Hofstetter, Alfons, Prof.Dr.med., 82008 Unterhaching (DE); Dühmke, Eckhart, Prof., 82327 Tutzing (DE); Schaffer, Moshe, 81377 Munich (DE); Jori, Giulio, 35100 Padova (IT); Corti, Luigi, Prof., 35152 Padua (IT)
(74) Vertreter: Hofstetter, Alfons J., Dr.rer.nat.

(57) **Zusammenfassung**

Die Erfindung betrifft die Verwendung von Photofrin als Radiosensitizer bei der Behandlung von tumorösem Gewebe, wobei nach einem vorbestimmten Zeitintervall nach Applikation des Photofrins eine Bestrahlung des tumorösen Gewebes mit ionisierender Strahlung erfolgt.

## Beschreibung

Die Erfindung betrifft die Verwendung von Photofrin als Radiosensitizer bei der Behandlung von tumorösem Gewebe.

Zur Behandlung von malignen Neoplasien wird häufig eine Photodynamische Therapie (PDT) eingesetzt. Die PDT beinhaltet die Verabreichung einer photosensibilisierenden Substanz und deren Aktivierung mit Licht geeigneter Wellenlänge, was zum Absterben der photosensibilisierten Zellen führt. Unter anderem werden auch Porphyrine und ihre Derivate als photosensibilisierende Substanzen bei der PDT eingesetzt.

In der WO 94/17797 wird beispielsweise die Verwendung von Photofrin als photoaktivierbare Substanz bei der medikamentösen Behandlung einer proliferierenden Gelenkkrankheit eines Patienten beschrieben, wobei das Photofrin in dem Gelenkinnenhautgewebe akkumuliert. Die Bestrahlung des Gelenkinnenhautgewebes mit Licht einer photoaktivierenden Wellenlänge bewirkt dann die Zerstörung des Gewebes. Als "Photofrin" bzw. "Photofrin II" wird eine durch Gelausschlußchromatographie gereinigte Fraktion von Hematoporphyrin-Derivaten (HPD) bezeichnet, die für die photosensibilisierenden Eigenschaften verantwortlich ist. Unter Hematoporphyrin-Derivaten ist dabei eine chemische Präparation einer komplexen Porphyrinmischung zu verstehen, die in Tumorgeweben selektiv akkumuliert. Die genaue Zusammensetzung des Photofrins ist bisher nicht bekannt, hierzu siehe auch C.J. Byrne, L.V. Marshallsay und A.D. Ward (1990) "The composition of photofrin" *Journal of Photochemistry and Photobiology 6,* Seiten 13-27. Nachteilig an der in der WO 94/17797 beschriebenen PDT ist jedoch, daß die Bestrahlung und damit die Zerstörung des betroffenen Gewebes nur erfolgen kann, wenn ein Zugang zu diesem Gewebe möglich ist und das zu behandelnde Gewebe direkt mit Licht der entsprechenden Wellenlänge bestrahlt werden kann. Dies ist jedoch bei zahlreichen Tumorerkrankungen nicht der Fall.

In der US 5,257,970 wird ein PDT-Verfahren beschrieben, mit dem ein Versuch unternommen wird, diesen Nachteil zu überwinden. Zusätzlich zu dem Photosensitizer werden dort Liposomen in einen Mensch oder ein Tier injiziert, die hitzesensibel sind und separate Komponenten eines Aktivierungssystems für die PDT einschließen. Die Liposomen werden anschließend selektiv an einem Tumor durch direkte Hitze produzierende Systeme zerstört, wodurch die Komponenten des Aktivierungssystems freigesetzt und gemischt werden. Dadurch wird eine Lichtemission und eine gleichzeitige Absorption durch die Photosensitizer erreicht. Alternativ kann auch ein anderer Energietransfer zu den vorher injizierten Photosensitizern oder eine Umsetzung zu einer cytotoxischen Spezies, die mit dem Tumor interagieren kann, erreicht werden. Als Folge wird der Tumor zerstört. Insgesamt scheint dieses Verfahren jedoch außerordentlich umständlich und in vielen Fällen nicht anwendbar.

In der medizinischen Literatur sind bereits Hinweise darauf zu finden, daß Porphyrine auch als Radiosensitizer verwendet werden können, wobei die hierzu benötigte Strahlung ionisierende Strahlung wie etwa Röntgenstrahlung ist, die den Körper ohne weiteres durchdringt und daher nicht durch ein zusätzliches Leitelement in die unmittelbare Nähe des zu bestrahlenden Gewebes geleitet werden muß. Eine deutliche Erhöhung der Tumorradiosensibilität wird beispielsweise bei Rabdomyosarkomen durch den Einsatz von Hematoporphyrinen hervorgerufen (L. Choen und S. Schwartz (1966) "Modification of radiosensitivity by porphyrin II transplanted rabdomyosarcoma in mice" *Cancer Research* 26, Teil 1, Seiten 11769-1773; S. Schwartz, M. Krepios, J. Modelevsky, H. Freyholtz, R. Walters und L. Larson (1978) "Modification of radiosensitivity by porphyrins: studies of tumors and other systems" in *Diagnosis and Therapy of Porphyrias and Lead Intoxication* (Ed. M. Doss) Springer Verlag, Berlin Heidelberg New York, Seiten 227-235). Substanzdosis-Wirkungs-Abhängigkeiten zeigten jedoch, daß bei hoher Hematoporphyrin-Dosis auch eine Radioprotektion erfolgen kann. Eine derartige Radioprotektion erschwert jedoch eine geeignete Dosierung außerordentlich, da die Konzentration innerhalb des Tumorgewebes stark von dem Zustand des zu behandelnden Tumors abhängig und daher schwer abschätzbar ist. Dies führt dazu, daß diese Art der Therapie in einigen Fällen aufgrund der auftretenden Radioprotektion unwirksam bleibt.

Aufgabe der vorliegenden Erfindung ist es daher, eine vergleichsweise einfache Möglichkeit bereitzustellen, auch tumoröses Gewebe wirksam behandeln zu können, das aufgrund seiner Position nicht mit Licht bestrahlt werden kann.

Diese Aufgabe wird gelöst durch die Verwendung von Photofrin als Radiosensitizer bei der Behandlung von tumorösem Gewebe, wobei nach einem vorbestimmten Zeitintervall nach Applikation des Photofrins eine Bestrahlung des tumorösen Gewebes mit ionisierender Strahlung erfolgt.

Wie sich überraschenderweise gezeigt hat, kann durch eine Kombination aus Photofrin-Applikation und Bestrahlung eine Inhibierung des Tumorwachstums erreicht werden. Bei keiner der bisher untersuchten Photofrin-Dosierungen wurde eine Radioprotektion durch Photofrin festgestellt. Folglich werden die Bestandteile des Hematoporphyrins, die die Radioprotektion verursachen, sehr wahrscheinlich bei der Herstellung des Photofrins abgetrennt. Aufgrund dessen besitzt Photofrin im Gegensatz zu den bisher bekannten Radiosensitizem außerordentliche Vorteile bei der Tumorbehandlung. Photofrin ist ein äußerst selektiver Radiosensitizer, da es sich im Tumorgewebe anreichert und die Photofrin-Konzentration im Tumorgewebe folglich wesentlich höher ist als in den übrigen Geweben. Durch die Verwendung von ionisierender Strahlung kann auch unzugängliches Tumorgewebe erfolgreich behandelt werden und in seinem Wachstum inhibiert werden. Zusätzlich zu der Photofrin-Applikation und der Bestrahlung mit ionisierenden Strahlen kann auch eine Hyperthermie-Therapie vorgenommen werden, die einen synergistischen Effekt hervorruft.

Besonders vorteilhaft ist eine zusätzliche Markierung des Photofrins mit einem Neutroneneinfänger. Eine solche Neutronen-einfangende Wirkung hat Gadolinium. Die Verwendung von Gadolinium-markiertem Photofrin ermöglicht nicht nur eine überaus effektive Therapie durch Neutronenstrahlung sondern auch eine präzise Diagnostik durch Kernspinresonanz. Auf diese Weise können die Tumorposition und die Tumorform genau bestimmt werden, wodurch eine äußerst zielgerichtete Behandlung des Tumors ermöglicht wird. Photofrin kann auch mit Bor markiert werden, um eine herkömmliche Bor-Neutroneneinfang-Therapie (boron neutron capture therapy, BNCT) durchführen zu können. Das mit Bor markierte Photofrin reichert sich wie Photofrin alleine oder auch Gadolinium-markiertes Photofrin in tumorösen Geweben an. Äußerst günstig erweist sich diese Therapie bei Hirntumoren.

Die verwendete ionisierende Strahlung kann Photonen- und/oder Elektronen- und/oder Gammaund/oder Alpha- und/oder Beta- und/oder Neutronen und/oder Protonenstrahlung sein. Dabei können gleichzeitig auch verschiedene Therapieformen bzw. Bestrahlungsmethoden gewählt werden, wie beispielsweise interstitielle Brachytherapie, stereotaktische Bestrahlung, Gamma-Messer-Radiochirurgie (Gamma Knife radiosurgery) und äußerliche bzw. perkutane Bestrahlung. Während die physikalische Wirkung der Porphyrine als Photosensitizer durch Absorption von Licht in einem Wellenlängenbereich von 350-900 nm erreicht wird, werden zwei theoretische Ansätze zur Erklärung des Wirkungsmechanismus der Porphyrine als Radiosensitizer derzeit diskutiert. Erstens könnte die Radiosensibilität durch eine erhöhte Sauerstoffkonzentration im Tumorgewebe erreicht werden, die durch die hohe Porphyrinkonzentration im Tumorgewebe und die dadurch verbesserte Durchblutung des Gewebes verursacht wird. Ferner könnte die Bestrahlung mit ionisierender Strahlung zur Erzeugung freier Radikale führen, die ihrerseits Porphyrine aktivieren und somit eine Reihe enzymatischer Reaktion auslösen mit dem Resultat der Tumorvernichtung.

Die Bestrahlung kann mit einer Strahlendosis von 2 bis 20 Gy, insbesondere 5 bis 15 Gy, erfolgen.

Um eine Regeneration der Tumorzellen nach einer Bestrahlung zu verhindern, kann auch eine Fraktionierungsbestrahlung durchgeführt werden. Dabei können auch unterschiedliche Bestrahlungsarten kombiniert werden. Das im Tumorgewebe angereicherte Photofrin bleibt bis zu 72 h nach der Photofrin-Applikation im Tumorgewebe. Eine Fraktionierungsbestrahlung ist daher bei geeigneten zeitlichen Abständen zwischen den einzelnen Bestrahlungen wie etwa 12 h bis 24 h sehr effektiv. Um schnelle Regenerationsprozesse zu vermeiden, können auch kürzere zeitliche Abstände von 3 h bis 12 h, insbesondere von 4 h bis 6 h gewählt werden.

Besonders vorteilhafter Weise erfolgt die Applikation des Photofrins intravenös. Auf diese Weise wird eine äußerst schnelle Verteilung des Photofrins im Organismus erreicht, die dann zur Akkumulation des Photofrins im Tumorgewebe führt.

Besonders wirkungsvoll ist das Photofrin, wenn es in einer Dosis von 7,5 mg bis 15 mg pro kg Körpergewicht intravenös injiziert wird.

Äußerst zweckmäßig erscheint weiterhin, daß das Zeitintervall zwischen der Applikation des Photofrins und der Bestrahlung zwischen 12 h und 48 h, insbesondere jedoch bei 24 h liegt. Innerhalb dieses Zeitintervalls erfolgt eine ausreichende Anreicherung des Photofrins im Tumorgewebe. Ein Zeitintervall von weniger als 12 h kann gewählt werden, um auch bei geringeren Photofrinkonzentrationen im Tumorgewebe eine Hemmung des Tumorwachstums zu erreichen.

Das Photofrin kann auch ein Bestandteil eines Arzneimittels zur Behandlung von tumorösem Gewebe sein. Auf diese Weise kann die Wirkung des Photofrins mit günstigen Eigenschaften anderer Substanzen kombiniert werden. Das tumoröse Gewebe kann dabei sowohl menschliches als auch tierisches Gewebe sein.

Weitere Vorteile der Erfindung gehen aus den im folgenden aufgeführten Untersuchungen hervor.

In einer ersten Untersuchung wurden als Tiermodelle weibliche Mäuse mit jeweils einem Körpergewicht von 20-22 g verwendet. Diesen Mäusen wurde jeweils subkutan ein Lewis-Karzinom transplantiert. Bei unbehandelten Mäusen wuchs der transplantierte Tumor äußerst schnell und verzwölffachte sein Volumen innerhalb von sechs Tagen. Das Tumorvolumen wurde täglich mit einem Taster bzw. einer Mikrometerschraube gemessen.

Photofrin wurde in einer therapeutischen Dosis von 0,5 oder 5 mg pro kg Körpergewicht in die Schwanzvene injiziert. Die Bestrahlung erfolgte mit 3 Gy 24 h nach der intravenösen Injektion des Photofrins. Die 30 verwendeten Mäuse wurden in fünf verschiedene gleich große Gruppen eingeteilt. Die Mäuse der Gruppe A wurden weder mit Photofrin behandelt noch bestrahlt. Die Mäuse der Gruppe B wurden ausschließlich mit 3 Gy bestrahlt. Den Mäusen der Gruppe C wurden jeweils 5 mg Photofrin pro kg Körpergewicht injiziert, ohne daß eine Bestrahlung folgte. Die Mäuse der Gruppe D wurden nach der Injektion von Photofrin von 5 mg pro kg Körpergewicht mit 3 Gy bestrahlt. Die Mäuse der Gruppe E wurden nach der Injektion von 0,5 mg Photofrin pro kg Körpergewicht mit ebenfalls 3 Gy bestrahlt.

**Tabelle 1**

| **Tumorgröße (cm**^{**3**}**)** | | | | | |
|---|---|---|---|---|---|
| Tag | A | B | C | D | E |
| 0 | 0.09 | 0.11 | 0.09 | 0.12 | 0.06 |
| 1 | 0.11 | 0.17 | 0.13 | 0.14 | 0.12 |
| 2 | 0.17 | 0.24 | 0.18 | 0.17 | 0.18 |
| 3 | 0.26 | 0.37 | 0.29 | 0.21 | 0.33 |
| 4 | 0.44 | 0.61 | 0.50 | 0.30 | 0.65 |
| 5 | 0.67 | 0.90 | 0.69 | 0.49 | 0.86 |
| 6 | 1.20 | 1.29 | 1.15 | 0.65 | 1.20 |

### Zeichenerklärung

- A=: Kontrolle, nicht-injizierte und unbestrahlte Mäuse
- B=: Kontrolle, bestrahlte, aber nicht-injizierte Mäuse
- C=: Mäuse, denen Photofrin (5 mg/kg) injiziert wurde, die aber nicht bestrahlt wurden
- D=: Mäuse, denen Photofrin (5 mg/kg) injiziert wurde und die bestrahlt (3 Gy) wurden
- E=: Mäuse, denen Photofrin (0,5 mg/kg) injiziert wurde und die bestrahlt (3 Gy) wurden

Die oben aufgeführten Daten stellen den Durchschnitt der Meßwerte von den jeweiligen Gruppen dar. Der durchschnittliche Abweichungsfaktor überschritt in keinem der Experimente 0.03.

Wie aus der Tabelle 1 hervorgeht, hat nur die Kombination aus einer höheren Photofrin-Dosis von 5 mg pro kg Körpergewicht und Bestrahlung einen entscheidenden Effekt auf das Tumorwachstum. Nur in der Mäusegruppe D wurde die Tumorwachstumsrate nach und nach verringert. Nach sechs Tagen entsprach das Tumorvolumen lediglich 50% des durchschnittlichen Tumorvolumens der Mäusegruppe A, die als Kontrolle diente. Dieses Ergebnis ist äußerst signifikant und belegt eindeutig, daß Photofrin als Radiosensitizer wirkt.

In einer weiteren Untersuchung wurden als Tiermodelle nackte Mäuse mit einem Körpergewicht von 20-22 g und einem subkutan transplantierten Tumor "Menschlicher Blasenkrebs RT4" verwendet. Das Tumorvolumen, das auch in dieser Untersuchung täglich gemessen wurde, stieg innerhalb von elf Tagen um etwa 3,5 bis 3,8 mm³. Photofrin wurde in einer therapeutischen Dosis von 5 mg pro kg Körpergewicht oder in einer Dosis von 10 mg pro kg Körpergewicht in die Schwanzvene injiziert. Die Bestrahlung erfolgte 24 h nach der intravenösen Injektion des Photofrins mit 5 Gy. Jeweils fünf Mäuse bildeten eine Untersuchungsgruppe. Die Mäuse der Gruppe A wurden weder mit Photofrin behandelt noch bestrahlt. Die Mäuse der Gruppe B wurden ausschließlich mit 5 Gy bestrahlt. Den Mäusen der Gruppe C wurde 10 mg Photofrin pro kg Körpergewicht injiziert, ohne daß eine Bestrahlung folgte. Die Mäuse der Gruppe D wurden nach der Injektion von Photofrin von 5 mg pro kg Körpergewicht mit 5 Gy bestrahlt. Die Mäuse der Gruppe E wurden nach der Injektion von 10 mg Photofrin pro kg Körpergewicht mit ebenfalls 5 Gy bestrahlt. Die Gruppe B bestand nach dem sechsten Tag nur noch aus vier Mäusen, da eine Maus starb.

Die Ergebnisse der Untersuchung sind in Tabelle 2 aufgeführt.

**Tabelle 2**

| **Tumorgröße (mm**^{**3**}**)** | | | | | |
|---|---|---|---|---|---|
| Tag | A | B | C | D | E |
| 0 | 3.42 | 3.46 | 3.42 | 3.46 | 3.64 |
| 1 | 3.46 | 3.54 | 3.66 | 3.5 | 3.72 |
| 2 | 3.72 | 3.64 | 3.84 | 3.58 | 3.82 |
| 3 | 4.02 | 3.86 | 4.12 | 3.72 | 3.92 |
| 4 | 4.46 | 4.34 | 4.78 | 3.92 | 4.02 |
| 5 | 5.14 | 5.2 | 5.7 | 4.18 | 4.22 |
| 6 | 5.98 | 5.65 | 6.5 | 4.58 | 4.36 |
| 7 | 7.0 | 6.7 | 8.24 | 5.08 | 4.6 |
| 8 | 8.04 | 7.49 | 9.62 | 5.52 | 4.78 |
| 9 | 9.42 | 8.67 | 11.0 | 5.98 | 5.16 |
| 10 | 10.74 | 10.9 | 12.64 | 6.54 | 5.5 |
| 11 | 12.96 | 13.63 | 14.27 | 7.5 | 5.9 |

### Zeichenerklärung

- A=: Kontrolle, nicht-injizierte und unbestrahlte Mäuse
- B=: Kontrolle, bestrahlte (5 Gy), aber nicht-injizierten Mäuse
- C=: Mäuse, denen Photofrin (10 mg/kg) injiziert wurde
- D=: Mäuse, denen Photofrin (5 mg/kg) injiziert wurde und die bestrahlt (5 Gy) wurden
- E=: Mäuse, denen Photofrin (10 mg/kg) injiziert wurde und die bestrahlt (5 Gy) wurden

Bei einer Bestrahlung mit 5 Gy (Gruppe B) konnte eine Inhibierung des Tumorwachstums bis zum neunten Tag festgestellt werden, während anschließend ähnliche Wachstumsraten wie bei der Kontrollgruppe A zu beobachten waren. Photofrin alleine hatte keinen Einfluß auf das Tumorwachstum im Vergleich zur Kontrollgruppe (siehe Gruppe C). Die Ergebnisse der Gruppen D und E zeigen, daß durch die Injektion von Photofrin das Tumorwachstum nach und nach verlangsamt wurde. Insgesamt betrug das Tumorvolumen bei den Kontrollgruppen am 11. Tag das 1,8fache des Tumorvolumens der Gruppe D und das 2,3fache des Tumorvolumens der Gruppe E. Die höhere Photofrin-Dosis von 10 mg pro kg Körpergewicht war folglich effizienter (siehe Gruppe E).

In einer dritten Untersuchung wurden als Tiermodelle wiederum nackte Mäuse mit einem Körpergewicht von 20-22 g eingesetzt, die jeweils einen subkutan transplantierten Tumor vom Typ "Menschlicher Blasenkrebs RT4" aufwiesen. Jeweils sechs Mäuse bildeten eine Versuchsgruppe. Die Mäuse der Versuchsgruppe A dienten der Kontrolle. Sie wurden nicht bestrahlt und ihnen wurde auch keine Substanz injiziert. Den Mäusen der Versuchsgruppe B wurde 15 mg Photofrin pro kg Körpergewicht injiziert, ohne daß sie zusätzlich bestrahlt wurden. Den Mäusen der Versuchsgruppen C, D, E, F und G wurden jeweils 15 mg, 10 mg, 7,5 mg, 5 mg und 2,5 mg Photofrin pro kg Körpergewicht injiziert. Nach 24 h folgte eine Bestrahlung mit 5 Gy. Die Tumorgröße wurde täglich gemessen. Die Ergebnisse dieser Messungen sind in Tabelle 3 aufgeführt.

**Tabelle 3**

| **Tumorgröße (mm**^{**3**}**)** | | | | | | | |
|---|---|---|---|---|---|---|---|
| Tag | A | B | C | D | E | F | G |
| 0 | 2.9 | 2.86 | 2.96 | 2.91 | 2.86 | 2.85 | 2.92 |
| 1 | 3.0 | 2.9 | 2.99 | 2.98 | 2.93 | 2.97 | 2.98 |
| 2 | 3.18 | 3.05 | 3.04 | 3.01 | 3.0 | 3.17 | 3.21 |
| 3 | 3.81 | 3.38 | 3.17 | 3.12 | 3.1 | 3.41 | 3.58 |
| 4 | 4.64 | 3.87 | 3.01 | 3.27 | 2.93 | 3.74 | 4.05 |
| 5 | 4.8 | 3.96 | 3.49 | 3.5 | 3.5 | 3.9 | 4.72 |
| 6 | 5.97 | 5.31 | 3.96 | 3.34 | 3.77 | 4.32 | 5.59 |
| 7 | 6.97 | 6.42 | 3.96 | 4.0 | 4.07 | 4.72 | 6.65 |
| 8 | 7.17 | 7.65 | 4.3 | 4.37 | 4.4 | 5.24 | 7.92 |
| 9 | 9.54 | 8.83 | 4.7 | 4.83 | 4.43 | 5.87 | 9.17 |
| 10 | 11.24 | 10.55 | 5.18 | 5.28 | 5.22 | 6.55 | 10.81 |
| 11 | 13.1 | 12.63 | 5.85 | 5.74 | 5.73 | 7.65 | 11.31 |

### Zeichenerklärung:

- A=: Kontrolle, nicht-injizierte und unbestrahlte Mäuse
- B=: Mäuse, denen 15 mg Photofrin pro kg Körpergewicht injiziert wurde
- C=: Mäuse, denen Photofrin (15 mg/kg) injiziert wurde und die mit 5 Gy bestrahlt wurden
- D=: Mäuse, denen Photofrin (10 mg/kg) injiziert wurde und die bestrahlt (5 Gy) wurden
- E=: Mäuse, denen Photofrin (7,5 mg/kg) injiziert wurde und die bestrahlt (5 Gy) wurden
- F=: Mäuse, denen Photofrin (5 mg/kg) injiziert wurde und die bestrahlt (5 Gy) wurden
- G=: Mäuse, denen Photofrin (2,5 mg/kg) injiziert wurde und die bestrahlt (5 Gy) wurden

Zusammenfassend ist festzustellen, daß Photofrin besonders bei einer Dosis von 7,5 bis 15 mg pro kg Körpergewicht sehr gut als Radiosensitizer wirkt. In diesem Dosisbereich sind keine signifikanten Unterschiede bezüglich der Wirksamkeit festzustellen. Wird Photofrin in einer geringeren Dosis (2,5-5 mg/ kg Körpergewicht) eingesetzt, wirkt es auch als Radiosensitizeraber mit einem geringeren Erfolg. Photofrin besitzt auch bei den hier untersuchten hohen Dosierungen keinen radioprotektiven Effekt.

Photofrin kann als Radiosensitizer bis zu einer Dosis von 20-30 mg pro kg Körpergewicht eingesetzt werden. Eine höhere Dosis würde toxisch wirken. Außerdem können auch höhere Strahlendosen eingesetzt werden. Geeignete Dosen liegen im Bereich von 2 bis 20 Gy, besser 5 bis 15 Gy, insbesondere bei 10 Gy.

## Patentansprüche

1. Verwendung von Photofrin als Radiosensitizer bei der Behandlung von tumorösem Gewebe, wobei nach einem vorbestimmten Zeitintervall nach Applikation des Photofrins eine Bestrahlung des tumorösen Gewebes mit ionisierender Strahlung erfolgt.

2. Verwendung nach Anspruch 1,
**dadurch gekennzeichnet,**
**daß** das Photofrin mit einem Neutroneneinfänger markiert ist.

3. Verwendung nach Anspruch 2,
**dadurch gekennzeichnet,**
**daß** der Neutroneneinfänger Bor und/oder Gadolinium ist.

4. Verwendung nach einem der vorhergehenden Ansprüche,
**dadurch gekennzeichnet,**
**daß** die ionisierende Strahlung Photonen- und/oder Elektronen- und/oder Gammaund/oder Alpha- und/oder Beta- und/oder Neutronen- und/oder Protonenstrahlung ist.

5. Verwendung nach einem der vorhergehenden Ansprüche,
**dadurch gekennzeichnet,**
**daß** die Bestrahlung mit einer Strahlendosis von 2 bis 20 Gy, insbesondere 5 bis 15 Gy, erfolgt.

6. Verwendung nach einem der vorhergehenden Ansprüche,
**dadurch gekennzeichnet,**
**daß** die Bestrahlung eine Fraktionierungsbestrahlung ist.

7. Verwendung nach einem der vorhergehenden Ansprüche,
**dadurch gekennzeichnet,**
**daß** die Applikation des Photofrins intravenös, erfolgt.

8. Verwendung nach Anspruch 7,
**dadurch gekennzeichnet,**
**daß** Photofrin in einer Dosis von 7,5 mg bis 15 mg pro kg Körpergewicht intravenös injiziert wird.

9. Verwendung nach einem der vorhergehenden Ansprüche,
**dadurch gekennzeichnet,**
**daß** das Zeitintervall zwischen 12 h und 48 h, insbesondere bei 24 h liegt.

10. Verwendung nach einem der vorhergehenden Ansprüche,
**dadurch gekennzeichnet,**
**daß** Photofrin ein Bestandteil eines Arzneimittels zur Behandlung von tumorösem Gewebe ist.
